# EUROPÄISCHE PATENTSCHRIFT

(11) **EP 2 091 912 B1**
(45) Veröffentlichungstag und Bekanntmachung des Hinweises auf die Patenterteilung: **17.02.2016**
(21) Anmeldenummer: 07847119.0
(22) Anmeldetag: 06.11.2007
(51) Int. Cl.: C07C 263/10, C07C 265/14, B01F 5/00, B01J 19/26, B01F 15/00, B01J 4/00

(54) **VERFAHREN ZUR HERSTELLUNG VON ISOCYANATEN**
METHOD FOR THE PRODUCTION OF ISOCYANATES
PROCEDE DE PRODUCTION D'ISOCYANATES

(30) Priorität: 07.11.2006 EP 06123631
(43) Veröffentlichungstag der Anmeldung: 26.08.2009
(73) Patentinhaber: BASF SE, 67056 Ludwigshafen (DE)
(72) Erfinder: WÖLFERT, Andreas, 74906 Bad Rappenau (DE); DAISS, Andreas, 67146 Deidesheim (DE); MATTKE, Torsten, 67251 Freinsheim (DE)
(86) Internationale Anmeldenummer: PCT/EP2007/061931
(87) Internationale Veröffentlichungsnummer: WO 2008/055898

(56) Entgegenhaltungen:
- EP-A- 1 449 826
- EP-A- 1 555 248
- WO-A-2007/028715

## Beschreibung

Die vorliegende Erfindung betrifft ein Verfahren zur Herstellung von Isocyanaten.

Zur Herstellung von Isocyanaten durch Phosgenierung der korrespondierenden Amine besteht prinzipiell die Möglichkeit einer Flüssigphasen- oder einer Gasphasenphosgenierung. Die Gasphasenphosgenierung zeichnet sich dadurch aus, daß die Reaktionsbedingungen so gewählt werden, daß zumindest die Reaktionskomponenten Diamin, Diisocyanat und Phosgen, bevorzugt jedoch sämtliche Edukte, Produkte und Reaktionszwischenprodukte, bei diesen Bedingungen gasförmig sind. Die vorliegende Erfindung betrifft ausschließlich die Gasphasenphosgenierung.

EP 1 275 639 A1 beschreibt die Gasphasenphosgenierung von (cyclo)aliphatischen Diaminen in einer Reaktionszone mit Einengungen der Wände.

In der Mischeinrichtung werden die amin- und phosgenhaltigen Eduktströme koaxial einer Mischzone zugeführt, wobei der phosgenhaltige Eduktstrom innen und der aminhaltige Eduktstrom außen geführt wird. Im Bereich der Zusammenführung der Eduktströme erfolgt eine weitere Reduzierung oder leichte Vergrößerung des Strömungsquerschnittes, so daß aufgrund der Volumenzunahme im Laufe der Reaktion die Strömungsgeschwindigkeit ansteigt.
Nachteilig bei dieser Anordnung ist, dass der Aminstrom koaxial außen geführt wird. Dadurch kann es zur Feststoffbildung an den Wänden der Mischeinrichtung kommen, da an den Wänden das Amin gegenüber dem Phosgen im Überschuss vorliegt, was die Nebenproduktbildung begünstigt. Ein weiterer Nachteil des Verfahrens ist, dass bei einer zu starken Beschleunigung der Strömung durch die Querschnittsverengung es zu einer Dämpfung der turbulenten Schwankungsgeschwindigkeiten in der Strömung kommt, die für das schnelle Mischen in einer turbulenten Strömung maßgeblich sind.

Ebenfalls in EP 1275639A1 wird beschrieben, dass vor der Zusammenführung der Eduktströme eine Verdrallung der Eduktströme erfolgen soll, so dass die turbulenten Schwankungsgeschwindigkeiten in den Eduktströmen erhöht sind und die Vermischung bei der Zusammenführung der beiden Eduktströme dann rascher erfolgt. Nachteilig ist allerdings, dass durch die Erhöhung der Schwankungsgeschwindigkeiten auch die Stromaufdispersion an der Mündung des aminhaltigen Eduktstromes in die Mischzone verstärkt wird und damit die Gefahr der Verstopfung der Mischdüse erhöht wird.

EP 1 275 640 A1 beschreibt die Gasphasenphosgenierung von (cyclo)aliphatischen Di- und Triaminen in einem Mischrohr mit Reaktor, in dem die Gasströmung im Mischbereich beschleunigt wird. Offenbart wird eine Reaktorgeometrie mit einem Innen- und einem Außenrohr, mit der eine Gasphasenphosgenierung im Maßstab von lediglich ca. 211 g Hexamethylendiamin-1,6 pro Stunde möglich ist.

Nachteilig bei diesem Verfahren ist, dass nicht sofort zu Beginn der Vermischung die maximale Geschwindigkeitsdifferenz zwischen den Reaktandenströmen erreicht wird und damit auch nicht die minimal mögliche Mischzeit erzielt wird.

Nachteilig an den in diesen beiden Schriften offenbarten Reaktoren ist, daß bei einem einfachen Übertragen dieser Reaktorgeometrie auf großtechnisch sinnvolle Größen der Durchmesser des Innenrohres so erhöht werden müßte, daß eine Durchmischung der durch die beiden Rohre dosierten Ströme aufgrund der langen quer zur Strömungsrichtung stehenden Strecken in kurzen Mischzeiten nicht mehr möglich ist.

In Patent EP 928785 A1 wird ein Verfahren zur Herstellung von Isocyanaten mit einer Mischeinrichtung beschrieben, die eine Vielzahl von Mikrostrukturmischelementen enthält. Nachteilig bei dem Verfahren sind jedoch die sehr kleinen freien Querschnitte der einzelnen Amin- und Phosgenzuführungen, die ganz besonders anfällig für die Ablagerung von Feststoffen und für Verstopfung sind.

In EP 1 449 826 A1 wird eine Vorverteilung des aminhaltigen Stromes auf mindestens zwei einzelne Amineinleitungen offenbart. Diese sind jedoch hydrodynamisch voneinander entkoppelt. Verringert sich der Querschnitt eines einzelnen dieser Rohre, beispielsweise durch Bildung der Aminhydrochloride, so wird automatisch der Durchsatz durch dieses Rohr wegen des Druckverlustanstiegs kleiner und führt zu einer verringerten Durchströmung. Kleinere Durchsätze führen aber dazu, dass sich noch mehr Feststoffe an der Wandung festsetzten, so dass die Verstopfung noch schneller fortschreitet. Nachteilig dabei ist, dass die Verweilzeitverteilung des Gemischstromes auf Grund des großen Querschnitt nach der Eindüsung bezogen auf die Reaktionszonenlänge relativ breit ist.

DE 10359627 A1 offenbart eine Gasphasenphosgenierung, in der Amin durch einen konzentrischen Ringspalt zwischen zwei Phosgenströme eingemischt wird, wobei die Flächen, durch die die Phosgenströme fließen, in einem Verhältnis von 1:0,5 bis 1:4 stehen.

Auch an dieser Reaktorgeometrie ist nachteilig, daß bei einem einfachen Übertragen dieser Reaktorgeometrie auf großtechnisch sinnvolle Größen der Innendurchmesser des Mischorgans nur bis zu einer gewissen Grenze erhöht werden kann, damit dieses Flächenverhältnis eingehalten werden kann.

WO 02/02217 beschreibt verschiedene Verfahren zur Vermischung von Eduktströmen, untern anderem für eine Flüssigphasenphosgenierung.

Nachteilig daran ist, daß die dort offenbarten Verfahren für die Vermischung von Flüssigphasen bei niedrigen Eintrittsgeschwindigkeiten von lediglich ca. 10 m/s konzipiert sind, wohingegen die bei Gasphasenphosgenierungen erforderlichen wesentlich höheren Geschwindigkeiten und die Vermischung von Gasen andere fluiddynamische Anforderungen stellen als die Vermischung von Flüssigkeiten. Zudem sind die Reaktionsgeschwindigkeiten einer Phosgenierung in der Gasphase deutlich von denen in der Flüssigphase verschieden, so daß das Verfahren aus der WO 02/02217 nicht ohne weiteres auf die Gasphase übertragbar ist.

Aus der WO 2007/028715 ist ein Verfahren bekannt, in dem Amin und Phosgen über Ringspalte, also ringförmig geschlossene Spalte dosiert werden.

Aufgabe der vorliegenden Erfindung war es nun, eine Reaktionsführung für eine Gasphasenphosgenierung zu entwickeln, mit der eine großtechnische Durchführung möglich wird und die mindestens einen der folgenden Vorteile bringt:
- Ermöglichung kurzer Mischungsweglängen
- keine oder nur eine sehr geringe Rückvermischung
- Unanfällig gegenüber Verstopfungen durch lediglich geringe Ablagerungsneigung von Feststoff
- günstige Verweilzeitverteilung

Die Aufgabe wird gelöst durch Verfahren zur Herstellung von Isocyanaten durch Umsetzung der korrespondierenden Amine mit Phosgen, gegebenenfalls in Gegenwart von mindestens einem, bevorzugt genau einem Inertmedium, in der Gasphase, in dem man in einem Reaktor n Aminströme mit mindestens n Phosgenströmen umgesetzt werden, wobei n eine positive ganze Zahl von mindestens 1 ist, und man mindestens einen Amin- oder Phosgenstrom über mindestens einen Schlitz zur Vermischung in den Reaktor dosiert, wobei man das Amin durch einen Schlitz dosiert, der an beiden Längsseiten von Schlitzen flankiert ist, durch die man Phosgen dosiert oder durch einen inneren Schlitz Amin dosiert, der allseitig von einem zusammenhängenden Schlitz umgeben ist, durch den man Phosgen dosiert und wobei die Flächenform sämtlicher Schlitze rechteckig ist.

In der WO 2007/028715 werden die Begriffe "Ringspalt" und "Ringspaltraum" wie folgt verwendet:

Der Ringspaltraum wird als eine Verallgemeinerung eines Hohlzylinders aufgefaßt (zur Definition des Hohlzylinders siehe Bronstein, "Taschenbuch der Mathematik", 21. Auflage, S. 199): Ein Ringspaltraum ist das Volumen, das von einer äußeren und einer inneren, jeweils räumlich gekrümmten, geschlossenen Mantelfläche und den begrenzenden Stirnflächen eingeschlossen ist, wobei sich die beiden Mantelflächen nicht gegenseitig durchdringen.

Der Ringspalt wird als Verallgemeinerung einer Kreisringfläche aufgefaßt (zur Definition der Kreisringfläche siehe Bronstein, "Taschenbuch der Mathematik", 21. Auflage, S. 194): Der Ringspalt hat die Form der Stirnfläche, die den oben beschriebenen Ringspaltraum an dessen Stirnseite begrenzt.

Demgegenüber handelt es sich bei dem Begriff "Schlitz" im Rahmen der vorliegenden Schrift um eine nicht-ringförmig-verbundene Fläche - stattdessen handelt es sich bei einem "Schlitz" um eine einfach zusammenhängende Fläche.

Derartige Schlitze können erfindungsgemäß beliebig geformt sein, solange sie nicht ringförmig verbunden sind. Beispiele für beliebige Formen sind lineare, wellenförmige, zick-zack-förmige oder schleifenförmige Schlitze, bevorzugt sind die Schlitze linear.

Die im Verfahren in den Mischraum zu dosierenden phosgen- und aminhaltigen Eduktströme treten auf einer Seite durch mindestens (2n), beispielsweise zwei flächige Schlitze in den Mischraum ein. Denkbar sind dabei verschiedene mögliche Ausführungsformen:

Die Schlitze können stapelförmig übereinander angeordnet sein. In diesem Fall ist beispielsweise ein Schlitz, durch den Amin dosiert wird, an beiden Längsseiten von bevorzugt parallel laufenden Schlitzen flankiert, durch die Phosgen dosiert wird. Diese können wiederum in der gleichen Weise von zwei Schlitzen zur Amindosierung umgeben sein, die wiederum von zwei Schlitzen flankiert sein können, durch die Phosgen dosiert wird.

In diesem Fall ergibt sich ein Schlitzstapel von (2n + 1) Schlitzen, wobei bevorzugt durch die äußeren Schlitze Phosgen dosiert wird.

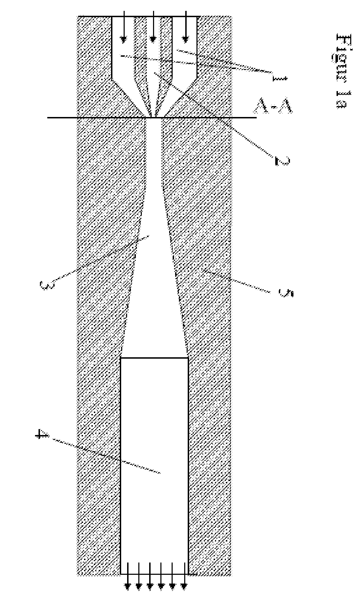

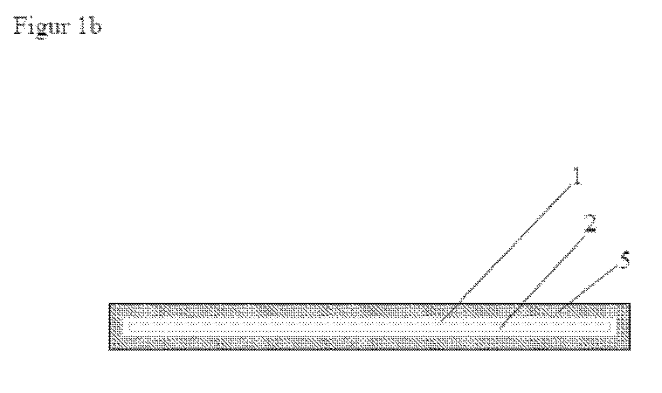

In einer weiteren Ausführungsform wird durch einen inneren Schlitz Amin dosiert, der allseitig von einem zusammenhängenden, beispielsweise rechteckigen Schlitz, umgeben ist, durch den Phosgen dosiert wird (Figuren 1 a und 1 b). Dieser kann dann beispielsweise von einem weiteren zusammenhängenden oder nicht-zusammenhängenden Schlitz zur Amindosierung flankiert sein, der wiederum allseitig von einem zusammenhängenden, beispielsweise rechteckigen Schlitz, umgeben ist, durch den Phosgen dosiert wird.

In diesem Fall ergibt sich eine bevorzugt Anordnung, in der ein Schlitz durch je einen zusammenhängenden Schlitz umgeben ist.

Zur Vermeidung von Feststoffablagerung und Verstopfungen wird bei der erfindungsgemäßen Mischeinrichtung der phosgenhaltige Eduktstrom bevorzugt so geführt, dass sämtliche Apparatewandungen nach Zusammenführung der Eduktströme von dem oder den phosgenhaltigen Eduktströmen überströmt werden und der oder die aminhaltigen Eduktströme vollständig von dem oder den phosgenhaltigen Eduktströmen solange umhüllt werden, bis eine vollständige Vermischung der Ströme oder ein weitgehend vollständiger Umsatz des Amins erfolgt ist.

Daher wird bevorzugt das Amin innen über einen Schlitz dosiert, der vollständig, also nicht nur oben und unten, sondern auch an den Seiten, von einem Phosgenstrom umgeben ist. Dieser umgebende, zusammenhängende Schlitz ist dann der Form des Schlitzes angepaßt, also beispielsweise bei einem linearen Schlitz zur Amindosierung durch eine rechteckige Dosierung für Phosgen (siehe Figur 1 b).

Die Amine, die in eine Gasphasenphosgenierung eingesetzt werden können, müssen bestimmte Erfordernisse erfüllen (siehe unten).

Dabei kann es sich um Monoamine, Diamine, Triamine oder höherwertige Amine handeln, bevorzugt um Diamine. Ensprechend ergeben sich die korrespondierenden Monoisocyanate, Diisocyanate, Triisocyanate oder höherwertigen Isocyanate, bevorzugt Diisocyanate.

Die Amine und Isocyanate können aliphatisch, cycloaliphatisch oder aromatisch sein, bevorzugt aliphatisch oder cycloaliphatisch und besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate stehen im Rahmen dieser Anmeldung kurz für cycloaliphatische und/oder aliphatische Isocyanate.

Beispiele für aromatische Diisocyanate sind bevorzugt solche mit 6 - 20 C-Atomen, beispielsweise monomeres 2,4'- oder 4,4'-Methylen-di(phenylisocyanat (MDI), 2,4- und/oder 2,6-Toluylendiisocyanat (TDI) und 1,5- oder 1,8-Naphtyldiisocyanat (NDI).

Diisocyanate sind bevorzugt (cyclo)aliphatische Diisocyanate, besonders bevorzugt (cyclo)aliphatische Diisocyanate mit 4 bis 20 C-Atomen.

Beispiele für übliche Diisocyanate sind aliphatische Diisocyanate wie 1,4-Tetramethylendiisocyanat, 1,5-Pentamethylendiisocyanat, Hexamethylendiisocyanat (1,6-Diisocyanatohexan), 1,8-Octamethylendiisocyanat, 1,10-Decamethylendiisocyanat, 1,12-Dodecamethylen-diisocyanat, 1,14-Tetradecamethylendiisocyanat, Derivate des Lysindiisocyanates, Tetramethylxylylendiisocyanat (TMXDI), Trimethylhexandiisocyanat oder Tetra-methylhexandiisocyanat, sowie 3 (bzw. 4), 8 (bzw. 9)-Bis(isocyanatomethyl)-tricyclo[5.2.1.0^{2.6}]decan-Isomerengemische, sowie cycloaliphatische Diisocyanate wie 1,4-, 1,3- oder 1,2-Diisocyanatocyclohexan, 4,4'- oder 2,4'-Di(isocyanatocyclo-hexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,5-Pentamethylendiisocyanat, 1,6-Diisocyanatohexan, 1-Isocyanato-3,3,5- trimethyl-5-(isocyanato-methyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanat-ocyclohexyl)methan.

Für das erfindungsgemäße Verfahren können solche Amine zur Reaktion zu den korrespondierenden Isocyanaten eingesetzt werden, bei denen das Amin, deren korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 mol%, besonders bevorzugt zu höchtens 1 mol% und ganz besonders bevorzugt zu höchstens 0,5 mol% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,5-Diaminopentan, 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (mol/mol) Gemisch, Diaminobenzol, 2,6-Xylidin, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-TDA.

Bei der Gasphasenphosgenierung ist es definitionsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCl) bilden, da die resultierenden Aminhydrochloride leicht ausfallen und nur schwer wieder verdampfbar sind.

Die Edukte, oder auch nur eines von ihnen, können zusammen mit mindestens einem Inertmedium in den Mischraum eindosiert werden.

Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Chlortoluol, o-Dichlorbenzol, Toluol, Xylol, Chlornaphthalin, Decahydronaphthalin, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,01 bis 15, besonders bevorzugt mehr als 0,1 bis 5 beträgt.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels durch die Mischeinrichtung dem Reaktor zugeführt.

Das bei der Phosgenierung verwendete Phosgen wird vor Durchführung des erfindungsgemäßen Verfahrens gegebenenfalls verdünnt mit einem Inertgas oder mit den Dämpfen eines inerten Lösungsmittels ebenfalls auf eine Temperatur innerhalb des Bereichs von 200°C bis 600°C, vorzugsweise 300°C bis 500°C erhitzt.

In einer bevorzugten Ausführungsform werden die n Aminströme auf eine bis zu 50 °C höhere Temperatur als die (n+1) Phosgenströme erhitzt, bevorzugt auf eine bis zu 30 °C, besonders bevorzugt bis zu 24 und ganz besonders bevorzugt bis zu 20 °C höher. Bevorzugt liegt die Temperatur der n Aminströme mindestens 5 °C, besonders bevorzugt mindestens 10 °C über der der (n+1) Phosgenströme.

Erfindungsgemäß wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Die Vermischung und Reaktion der beiden gasförmigen Edukte findet nach dem erfindungsgemäßen Verfahren nach der Einleitung der Eduktströme Diamin und Phosgen über die Schlitze als Eintrittsflächen in dem Mischraum als Reaktionsraum statt.

Die Reaktion setzt in der Regel mit Kontakt der Edukte unmittelbar nach der Vermischung ein.

So findet im vorderen Teil des Reaktionsraums die Vermischung der Edukte, gegebenenfalls vermischt mit Inertmedium, statt (Mischraum).

Zur Durchführung der erfindungsgemäßen Umsetzung werden der vorerhitzte Strom enthaltend Amin oder Gemische von Aminen und der vorerhitzte Strom enthaltend Phosgen kontinuierlich in den Reaktor, bevorzugt einen Rohrreaktor geleitet.

Die Reaktoren bestehen im allgemeinen aus Stahl, Glas, legiertem oder emaillierten Stahl und weisen eine Länge auf, die ausreichend ist, um unter den Verfahrensbedingungen eine vollständige Umsetzung des Diamins mit dem Phosgen zu ermöglichen.

### Mischeinrichtung

Die (n+1) Phosgenströme und n Aminströme werden im allgemeinen an einem Ende des Reaktors über eine Mischeinheit einem Verweilzeitreaktor zugeführt. Zwischen jeweils 2 Phosgenströme wird über einen Schlitz ein Aminstrom mit hoher Geschwindigkeit zugeführt. Die Zuführung aller Phosgenströme und Aminströme erfolgt jeweils über Schlitze in einem Schlitzstapel. Bevorzugt ist jeder Schlitz von einer entsprechend angepaßten Dosierungsmöglichkeit für die andere Reaktionskomponente umgeben (siehe Figur 1 b). Dabei handelt es sich bei den Schlitzen, mit denen benachbarte Phosgenströme und der dazwischen liegende Aminstrom zugeführt wird, bevorzugt um solche, bei denen der innere Aminstrom vollständig von einem Phosgenstrom umgeben ist.

In einer Ausführungsform können diese dann von wiederum konzentrisch von weiteren Dosierungsmöglichkeiten umgeben sein.

Die Schlitze weisen bevorzugt ein Verhältnis von Breite zu Höhe von mindestens 2 : 1, bevorzugt mindestens 3:1, besonders bevorzugt mindestens 4:1, ganz besonders bevorzugt mindestens 5:1 und insbesondere mindestens 10:1 auf. Nach oben wird das Verhältnis von Breite zu Höhe lediglich durch konstruktiv sinnvolle und praktikable Werbegrenzt. In der Regel beträgt das Verhältnis weniger als 500:1, bevorzugt weniger als 250:1 und besonders bevorzugt weniger als 100:1.

Breite und Höhe sind dabei jeweils bezogen auf größte Breite bzw. die größte Höhe des Schlitzes.

Die Flächenform der Schlitze ist rechteckig.

Es kann sinnvoll sein, in den Eduktzuleitungen Strömungsvergleichmäßiger einzubauen, wie sie beispielsweise aus der EP 1362847 A bekannt sind. Bevorzugt wird zur Vergleichmäßigung der Geschwindigkeit der Eduktströme jedoch eine im Verhältnis zum Durchmesser der Zuleitung lange Vorlauflänge in der Eduktzuleitung, das 2 bis 40-fache des Zuleitungsdurchmessers, besonders bevorzugt das 4 bis 30-fache, ganz besonders bevorzugt das 5 bis 20-fache.

Eine wie in Patent EP 1275640A1 beschriebene Verengung des Strömungsquerschnittes nach der Zusammenführung der Eduktströme zur Vermeidung von Rückstömungen ist möglich, bevorzugt kann jedoch darauf verzichtet werden. Erfindungsgemäß entscheidend für die Durchmischung der Ströme ist eine möglichst kleine Querdiffusionsstrecke, über die die Fluidelemente sich durch turbulente und laminare Diffusion austauschen und so die Mischung bewirken. Während in der DE 103 59 627 A1 der Aminstrom über ein einen Ringspalt bildendes doppelwandiges Leitrohr dosiert wird, wird von den beiden Phosgenströmen lediglich der äußere über einen Ringspalt dosiert, nämlich über eine "Querschnittsfläche, die von der Wand des Rohrreaktors und der äußeren Wand des doppelwandigen Leitrohrs begrenzt wird", wohingegen der innere Phosgenstrom über die "Querschnittsfläche, die von der inneren Wand des doppelwandigen Leitrohrs begrenzt wird" dosiert wird, also über eine kreisförmige Fläche. Somit nimmt, der Lehre der DE 103 59 627 A1 folgend, bei einer Vergrößerung des Querschnitts des Reaktors bzw. der inneren Phosgendosierung Querdiffusionsstrecke zwischen dem inneren Phosgenstrom und dem Aminstrom zu. Erfindungsgemäß wird der Aminstrom über einen Schlitz zwischen zwei ihrerseits über Schlitze dosierte Phosgenströme gemischt. Dieses Erfindungsprinzip ist analog auch für die Dosierung von n Aminströmen, beispielsweise 2, 3, 4 oder mehr, zwischen jeweils zwei Lagen von (n + 1) Phosgenströmen verallgemeinerbar.

Bevorzugt ist n 1, 2 oder 3, besonders bevorzugt 1 oder 2 und ganz besonders bevorzugt ist n = 1.

Damit ein Aminstrom dem Erfindungsgedanken folgend nach Beginn der Vermischung keinen Kontakt mit den Apparatewandungen hat, sondern von phosgenhaltigen Eduktströmen umhüllt ist, wird der Aminstrom "sandwichartig" zwischen zwei Phosgenströme dosiert.

Dies bedingt erfindungsgemäß, daß es sich bei dem obersten und dem untersten bzw. dem äußersten Strom jeweils um einen Phosgenstrom handelt, der den oder die Aminströme von den Wandungen des Reaktors abhält.

Die Strömungsquerschnitte des oder der phosgenhaltigen Eduktströme werden so gestaltet, dass das charakteristische Mischungslängenmaß wieder möglichst klein wird. Da das Edukt Phosgen im stöchiometrischen Überschuss zu geführt wird und außerdem die Phosgengeschwindigkeit bevorzugt kleiner als die Amingeschwindigkeit ist, muss eine größere Querschnittsfläche als für den aminhaltigen Strom gewählt werden, woraus sich auch größere charakteristische Abmaße ergeben. Die Mischungsweglänge wird kleiner als 200 mm, bevorzugt kleiner als 100 mm, besonders bevorzugt kleiner als 50 mm, ganz besonders bevorzugt kleiner als 25 mm und insbesondere kleiner als 10 mm gewählt. Die Mischungsweglänge ist dabei als die Distanz definiert, die Fluidelemente zweier oder mehrerer Eduktströme senkrecht zur Fliessrichtung der Eduktströme maximal zurücklegen müssen bis eine molekulare Vermischung der Eduktströme erfolgt ist.

Das Verhältnis der Gesamtfläche der Aminströme zur Gesamtfläche der Phosgenströme ist größer als 0,00002, bevorzugt größer als 0,0002, besonders bevorzugt größer als 0,002 und ganz besonders bevorzugt größer als 0,02.

Das Verhältnis der Gesamtfläche der Aminströme zur Gesamtfläche der Phosgenströme ist kleiner als 5, bevorzugt kleiner als 1, besonders bevorzugt kleiner als 0,5 und ganz besonders bevorzugt kleiner als 0,2.

Das Flächenverhältnis zweier phosgenführender Flächen, die durch einen aminführenden Schlitz getrennt sind, beträgt 0,1 bis 10, bevorzugt 0,2 bis 5, besonders bevorzugt 0,4 bis 2,5, ganz besonders 0,8 bis 1,25, insbesondere 0,9 bis 1,1 und speziell 1.

Bei n≥2 beträgt das Flächenverhältnis zweier aminführender Flächen, die durch eine phosgenführende Fläche getrennt sind, von 0,1 bis 10, bevorzugt 0,2 bis 5, besonders bevorzugt 0,4 bis 2,5, ganz besonders 0,8 bis 1,25, insbesondere 0,9 bis 1,1 und speziell 1.

Da die Intensität und Schnelligkeit der Vermischung der amin- und phosgenhaltigen Eduktströme wesentlich vom sich in der Mischzone einstellenden Schergradienten abhängen, muss die Mischzone so gestaltet werden, dass der Schergradient besonders groß ist.

Dazu sollte einerseits die Differenzgeschwindigkeit zwischen den amin- und phosgenhaltigen Eduktströmen besonders hoch gewählt werden und zum anderen die charakteristischen Längenabmaße möglichst klein gewählt werden, da der Schergradient proportional zum Quotienten aus Geschwindigkeitsdifferenz und charakteristischem Längenmaß ist.

Da die Geschwindigkeitsdifferenz zwischen den amin- und phosgenhaltigen E-duktsströmen hoch sein soll, müssen entweder die phosgenhaltigen oder die aminhaltigen Eduktströme eine größere Geschwindigkeit aufweisen. Da die Aminzuführungen zur Mischzone empfindlicher gegenüber der Bildung von Ablagerungen und Verstopfungen sind und eine Rückströmung in der Aminzuführung auf jeden Fall zu vermeiden ist, wird die Strömungsgeschwindigkeit des aminhaltigen Eduktstromes bevorzugt größer gewählt als die Geschwindigkeit des phosgenhaltigen Eduktstromes.
Je höher die Geschwindigkeit der aminhaltigen Eduktströme ist um so höher kann bei gleichem Schergefälle auch die Geschwindigkeit der phosgenhaltigen Eduktströme gewählt werden. Eine höhere Phosgengeschwindigkeit bewirkt kleinere Strömungsquerschnitte der Phosgenzuführung und damit kleinere Mischungsweglängen und somit ein schnelleres Mischen.
Um eine möglichst hohe Amingeschwindigkeit zu erreichen, wird daher angestrebt im Aminstrom am Punkt der Zusammenführung mit dem Phosgenstrom eine lokale Machzahl von größer als 0,6 einzustellen.

Die Machzahl bedeutet dabei das Verhältnis zwischen lokaler Strömungsgeschwindigkeit und lokaler Schallgeschwindigkeit. In einer besonderen Ausführungsform des Verfahrens wird die Zuführung des aminhaltigen Eduktströme so gewählt, dass am Austritt der Aminströme in die Mischzone gerade eine Machzahl von 1 vorliegt.

Im Fall einer sogenannten angepassten Aminzuführung entspricht der Druck des Aminstromes an diesem Punkt gerade dem Druck, des phosgenhaltigen Eduktstroms am Punkt der Zusammenführung. Im Falle einer nichtangepassten Aminzuführung ist der Druck des Aminströmes beim Austritt aus der Aminzuführung größer als der Druck des phosgenhaltigen Stromes bei der Zusammenführung. In diesem Fall kommt es dann zu einer weiteren Expansion des aminhaltigen Stromes, der mit einer Druckabsenkung bis auf den Druck des phosgenhaltigen Stromes verbunden ist.
Ob eine Düse angepasst oder nicht angepasst betrieben wird, hängt vom Vordruck des aminhaltigen- und des phosgenhaltigen Stromes vor der Mischdüse ab.
In einer weiteren besonderen Ausführungsform ist die Aminzuführung so gestaltet, dass bereits in der Zuführungen Machzahlen von größer 1 erreicht werden. Dies kann z.B. dadurch erreicht werden, dass die Zuführung des aminhaltigen Ströme in Form einer oder mehrer Lavaldüsen gestaltet wird, die sich dadurch auszeichnen, dass sich der Strömungsquerschnitt zunächst verengt bis eine Machzahl von eins erreicht ist und sich, dann wieder erweitert, was zu einer weiteren Expansion und Beschleunigung der Strömung führt. Um eine Überschallströmung (Machzahl größer als 1) zu erreichen, muss das Verhältnis des Aminkesseldruckes zum Mischzonendruck größere als das sogenannte kritische Druckverhältnis sein. Je höher das Druckverhältnis ist und je höher die Kesseltemperatur des Aminstromes ist, umso höher ist die maximal erreichbare Geschwindigkeit.
Da das Edukt Amin bei zu hohen Temperaturen oft thermisch geschädigt wird, dürfen allerdings keine zu hohen Temperaturen eingestellt werden. Auch kann der Aminvordruck auf Grund des Amindampfdruckes nicht beliebig gesteigert werden.
Bevorzugt wird die Aminzuführung daher so gestaltet, dass sich im aminhaltigen Eduktstrom direkt an der Zusammenführung mit dem phosgenhaltigen Strom oder im Falle einer nicht angepassten Düse kurz dahinter Machzahlen von 0,6 bis 4, besonders bevorzugt 0,7 bis 3, ganz besonders bevorzugt 0,8 bis 2,5 und insbesondere 0,9 bis 2,0 einstellen.
Die angegebenen Machzahlen kann der Fachmann bei bekannter Kesseltemperatur und bekannten Stoffdaten einfach in Strömungsgeschwindigkeiten umrechnen. Ebenso kann der Fachmann in Abhängigkeit von der angegebenen Machzahl und den Stoffdaten den erforderlichen Vordruck berechnen.
Die hohe Eintrittsgeschwindigkeit des Aminstromes in die Mischzone dient, wie oben dargestellt, der Erzielung einer möglichst großen Geschwindigkeitsdifferenz zwischen amin- und phosgenhaltigen Eduktströmen. Ferner wird durch die hohe Strömungsgeschwindigkeit der Systemdruck und damit auch die Eduktkonzentrationen sowie die Temperatur lokal reduziert, was zu einer Verringerung der Reaktionsgeschwindigkeiten und damit zu einer Vereinfachung der Mischaufgabe führt.
Um möglichst kleine Mischungsweglängen zu erreichen, muss angestrebt werden, die Strömungsgeschwindigkeit des phosgenhaltigen Eduktstromes ebenfalls möglichst hoch zu wählen, ohne jedoch die Differenzgeschwindigkeit zwischen amin- und phosgenhaltigem Eduktstrom zu sehr zu reduzieren. Dazu wird die Querschnittsfläche des Phosgenstromes so gewählt, dass sich eine Machzahl von 0,2 bis 2,0, bevorzugt 0,3 bis 1,5, besonders bevorzugt 0,4 bis 1,0, ganz besonders bevorzugt von 0,5 bis 1,0 und insbesondere 0,7 bis 1,0 ergibt.
Die Strömungsquerschnitte der aminhaltigen Eduktströme werden in der erfindungsgemäßen Mischeinheit so gestaltet, dass einerseits eine hohe Betriebstabilität gewährleistet ist und anders möglichst kleine Mischungsweglängen eingehalten werden. Daher werden für die Zuführung des aminhaltigen Eduktsstromes charakteristische Längenmaße von 0,5 bis 50 mm, bevorzugt 0,75 bis 25 mm, besonders bevorzugt 1 mm bis 10 mm und ganz besonders bevorzugt 1 mm bis 5 mm gewählt. Als charakteristisches Längenmaß ist dabei der kleinste Längenmaßstab des Strömungsquerschnitts gemeint, d.h. z.B. im Fall eines Spaltes die Spaltbreite oder im Fall einer kreisförmigen Öffnung der Öffnungsdurchmesser.

Bevorzugt werden die einzelnen Edukte in der Mischeinrichtung mit einer Strömungsgeschwindigkeit von 20 bis 400 Meter/Sekunde in den Reaktor geführt, bevorzugt von 25 bis 300 Meter/Sekunde, besonders bevorzugt 30 bis 250, ganz besonders bevorzugt 50 bis 200, insbesondere mehr als 150 bis 200 und speziell 160 bis 180 Meter/Sekunde.

In einer möglichen Ausführungsform der Erfindung kann es sinnvoll sein, die Phosgenströme, insbesondere den äußeren Phosgenstrom mit einer höheren Strömungsgeschwindigkeit in den Mischraum einzuführen, als den Aminstrom, den sie umhüllen, besonders bevorzugt mit mindestens 10 m/s mehr, ganz besonders bevorzugt mindestens 20 m/s mehr und insbesondere mindestens 50 m/s mehr.

Es kann aber auch möglich und sinnvoll sein, den äußeren Phosgenstrom mit einer höheren Strömungsgeschwindigkeit in den Mischraum einzuführen, als den Aminstrom, und den inneren Phosgenstrom mit einer niedrigeren Strömungsgeschwindigkeit. Dies stellt eine weitere mögliche Ausführungsform der vorliegenden Erfindung dar.

In einer bevorzugten Ausführungsform der Erfindung ist es sinnvoll, die Phosgenströme, insbesondere den äußere Phosgenstrom mit einer niedrigeren Strömungsgeschwindigkeit in den Mischraum einzuführen, als den Aminstrom, den sie umhüllen, besonders bevorzugt mit mindestens 50 m/s weniger, ganz besonders bevorzugt mindestens 60 m/s weniger, ganz besonders bevorzugt 80 m/s weniger und im speziellen mindestens 100 m/s weniger.

In einer bevorzugten Ausführungsform der vorliegenden Erfindung sind die (n+1) Schlitze der Phosgenströme mit genau einer Phosgenzuleitung druckverlustarm ohne zusätzliche Regeleinrichtungen verbunden, so daß die Geschwindigkeit, mit der das Phosgen aus den (n+1) Schlitzen strömt, etwa gleich ist.

Desgleichen gilt, daß die n Schlitze der Aminströme bevorzugt mit genau einer Aminzuleitung druckverlustarm ohne zusätzliche Regeleinrichtungen verbunden sind, so daß die Geschwindigkeit, mit der das Amin aus den n Schlitzen strömt, etwa gleich ist.

Es ist aber auch möglich, die Phosgen- und/oder Aminströme der Schlitze mit jeweils einer getrennt geregelten Zuleitung zu verbinden, so daß die Geschwindigkeiten pro Zuleitung einzeln und unabhängig voneinander einstellbar sind.

Die Edukte treten mit einem Geschwindigeitsvektor in den Mischraum. Dabei lässt sich der Geschwindigkeitsvektor in eine axiale, radiale und tangentiale Richtungskomponente unterteilen. Unter der axialen Richtung wird die Richtungskomponente des Geschwindigkeitsvektors parallel zur Längsachse des Mischraums verstanden. Unter der radialen Richtung wird die Richtungskomponente des Geschwindigkeitsvektors von aussen auf die Längsachse zu verstanden, also einen rechten Winkel mit der Längsachse einschließend. Unter tangentialer Richtung wird die Richtungskomponente des Geschwindigkeitsvektors parallel zur Berandung des Mischraums verstanden, also eine ringförmige Umlaufbewegung.

Zur Vermischung der Eduktströme läßt sich eine Verbesserung der sich einstellenden Vermischung durch den Einbau von eine Tangentialgeschwindigkeit erzeugenden Elementen erzielen, beispielsweise in die Zuleitung der Teilströme der Überschußkomponenten in den Mischraum. Ein geeignetes tangentialgeschwindigkeitserzeugendes Element wäre beispielsweise ein in die Zuleitung eingelassenes spiralförmig verdrilltes Band (Wendel), runde oder eckige Leitbleche (Leitschaufeln) oder dergleichen. Die Wirkung der tangentialgeschwindigkeitserzeugenden Einbauten ist es, in der Strömung der Düse die Scherung zwischen Strömungsschichten unterschiedlicher Zusammensetzung zu erhöhen.

Zur Erzeugung einer Tangentialgeschwindigkeit ist auch ein tangentialer Eintritt der Zuleitung eines oder mehrerer Eduktströme möglich oder bei einem radialen Zustrom eines oder mehrerer Eduktströme ein Schaufelkranz.

Weiterhin kann es sinnvoll sein, die Phosgen- und Aminströme mit gegenläufiger Tangentialgeschwindigkeit in den Mischraum einzuleiten, beispielsweise indem die Phosgenströme mit einer Tangentialgeschwindigkeit entlang der Längsachse des Reaktors blickend im Uhrzeigersinn, und der zwischenliegende Aminstrom mit einer Tangentialgeschwindigkeit gegen den Uhrzeigersinn in den Mischraum eindosiert werden.

Der Winkel, den der Summenvektor aus den Vektoren der Tangentialgeschwindigkeit und aus dem Vektor der Axialgeschwindigkeit der so eindosierten Ströme mit der Längsachse des Reaktors einschließt, kann von 5 bis 85°, bevorzugt 17 bis 73°, besonders bevorzugt 30 bis 60° für die einen Ströme, beispielsweise die Phosgenströme, und von -5 bis -85°, bevorzugt -17 bis --73°, besonders bevorzugt -30 bis -60° für die anderen Ströme, beispielsweise den Aminstrom betragen.

Weiterhin ist es sinnvoll, die Strömungen mit unterschiedlichen Radialgeschwindigkeiten in den Mischraum einzudosieren. Dabei stellt sich ein Winkel zwischen dem Summenvektor aus dem Radialgeschwindigkeitsvektor und aus dem Axialgeschwindigkeitsvektor mit der Längsachse ein. Dieser Winkel entspricht in der Regel dem Winkel des zugehörigen Dosierkanals mit der Längsachse des Mischraums. Dabei bedeutet eine negativer Winkel eine Dosierung von innen nach außen, ein positiver Winkel eine Dosierung von außen nach innen, ein Winkel von 0° bedeutet eine zur Längsachse des Mischraums parallele Strömung und ein Winkel von 90° eine zur Längsachse des Mischraums senkrechte Strömung.

Der äußere Phosgenstrom kann durch die Mischeinrichtung unter einem radialen Winkel von 0 bis 85°, bevorzugt 5 bis 85°, besonders bevorzugt 7 bis 65°, ganz besonders bevorzugt 15 bis 35° und insbesondere 18 bis 30° in den Mischraum eindosiert werden.

Der Aminstrom kann durch die Mischeinrichtung unter einem radialen Winkel von -50° bis +50°, bevorzugt -25 bis 25°, besonders bevorzugt -10 bis 10° und ganz besonders bevorzugt -3 bis +3° in den Mischraum eindosiert werden.

Der innere Phosgenstrom kann durch die Mischeinrichtung unter einem radialen Winkel von 0 bis -85°, bevorzugt -5 bis -85°, besonders bevorzugt -7 bis -65°, ganz besonders bevorzugt -15 bis -35° und insbesondere -18 bis -30° in den Mischraum eindosiert werden.

Vorteilhaft ist es, wenn äußerer Phosgenstrom und Aminstrom relativ zueinander einen radialen Winkel von 1 bis 60°, bevorzugt 7 bis 50, besonders bevorzugt 15 bis 45° und besonders bevorzugt 18 bis 35° einschließen.

Weiterhin vorteilhaft ist es, wenn Aminstrom und innerer Phosgenstrom relativ zueinander einen radialen Winkel von 1 bis 60°, bevorzugt 10 bis 50°, besonders bevorzugt 15 bis 45° und besonders bevorzugt 18 bis 35° einschließen.

Um einen möglichst vollständigen Umsatz des Amins zum jeweiligen Wertprodukt zu erreichen, wird mit den oben dargestellten Maßnahmen eine Mischzeit des phosgenhaltigen mit dem aminhaltigen Eduktstromes von kleiner 10 ms, bevorzugt kleiner 5 ms, besonders bevorzugt kleiner 2 ms, ganz besonders bevorzugt kleiner 1 ms und insbesondere kleiner 0,5 ms erreicht. Als Mischzeit wird dabei die Zeit definiert, die Fluidelemente, die aus der Aminzuführung austreten maximal benötigen, bis sich in ihnen ein Phosgen/Aminverhältnis von größer oder gleich 4 einstellt. Die Zeit zählt dabei jeweils ab dem Austritt eines Fluidelements aus der Aminzuführung.

### Reaktionsraum

Der Reaktionsraum umfaßt im vorderen Bereich den Mischraum, in dem überwiegend die Vermischung des gasförmigen Gemisches von Phosgen, Amin, gegebenenfalls vermischt mit Inertmedium, stattfindet, was in der Regel begleitet ist vom Einsetzen der Reaktion. Im hinteren Teil des Reaktionsraums findet dann im wesentlich nur noch die Reaktion statt und höchstens untergeordnet die Vermischung.

Zu Unterscheidungszwecken kann als Mischraum der Bereich des Reaktionsraumes bezeichnet werden, in dem die Vermischung der Edukte zu einem Grad von 99% stattfindet. In einer bevorzugten Ausführungsform der vorliegenden Erfindung ist der Umsatz im Mischraum, d.h. der Verbrauch des eingesetzten Amins, weniger als 15%. Dabei wird der Vermischungsgrad als Verhältnis der Differenz des lokal gemittelten Mischungsbruch und des Anfangsmischungsbruchs vor Vermischung zur Differenz des mittleren endgültigen Mischungsbruchs nach Vermischung und des Anfangsmischungsbruchs vor Vermischung angegeben. Zum Konzept des Mischungsbruches siehe z.B. J. Warnatz, U. Maas, R.W. Dibble: Verbrennung, Springer Verlag, Berlin Heidelberg New York, 1997, 2. Auflage, S. 134.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten moderaten Drücken standhalten. Geeignete Materialien für den Kontakt mit dem Reaktionsgemisch sind z.B. Metalle, wie Stahl, Tantal, Silber oder Kupfer, Glas, Keramik, Emaille oder homogene oder heterogene Gemische daraus. Bevorzugt werden Stahlreaktoren verwendet. Die Wände des Reaktors können hydraulisch glatt oder profiliert sein. Als Profile eignen sich beispielsweise Ritzen oder Wellen. Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42.

Bevorzugt verwendet werden Rohrreaktoren.

Ebenfalls ist es möglich, im wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Als technisch sinnvoll haben sich Reaktionsräume mit einem Verhältnis von Breite zu Höhe bis zu 5000 : 1, bevorzugt bis zu 1000 : 1 erwiesen.

Die Umsetzung von Phosgen mit Amin im Reaktionsraum erfolgt bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

In einer möglichen Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Druckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600 °C, bevorzugt bis zu 570 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt mehr als 0,01 Sekunden bis weniger als 3 Sekunden, besonders bevorzugt mehr als 0,015 Sekunden bis weniger als 2 Sekunden. Im Fall der Umsetzung von (cyclo)aliphatischen Aminen kann die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen.

Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im Reaktor des erfindungsgemäßen Verfahrens durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde.

Durch die turbulente Strömung werden enge Verweilzeitverteilungen mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Es kann sinnvoll sein, in den Reaktor Strömungsvergleichmäßiger einzubauen, wie sie beispielsweise aus der EP 1362847 A bekannt sind.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen.

In einer bevorzugten Ausführungsform werden die Umsetzungsbedingungen so gewählt, dass das Reaktionsgas am Austritt aus dem Reaktionsraum eine Phosgenkonzentration von mehr als 25 mol/m³, bevorzugt von 30 bis 50 mol/m³, aufweist. Weiterhin liegt am Austritt aus dem Reaktionsraum im allgemeinen eine Inertmediumskonzentration von mehr als 25 mol/m³, bevorzugt von 30 bis 100 mol/m³ vor.

Der Reaktionsraum kann konstruktiv im wesentlichen in bis zu vier Längenabschnitte entlang der Längsachse des Reaktors im Verlauf der Strömung zerlegt werden:
- ein erster, zumeist kurzer Abschnitt hinter der Eduktzuführeinrichtung mit der Länge L₁,
- ein zweiter Abschnitt mit der Länge L₂,
- ein dritter Abschnitt mit der Länge L₃ gefolgt von
- einem vierten Abschnitt mit der Länge L₄, der einen Rohrreaktor ohne inneren Verdrängerkörper darstellt und an den sich ein Quench (siehe unten) anschließt.

Als Reaktionsraum wird das Volumen beschrieben, in dem mindestens 98% des Umsatzes, d.h. der Verbrauch des eingesetzten Amins, stattfindet, bevorzugt mindestens 99%, besonders bevorzugt 99,5%, ganz besonders bevorzugt 99,7%, insbesondere 99,9% und speziell 99,99%.

Die einzelnen Parameter, die die Geometrien in diesen bis zu vier Abschnitten beschreiben, werden durch die Indize 1, 2, 3 und 4 bezeichnet.

Hauptsächlich charakterisierend sind als Parameter R für den Gesamtdurchmesser des Rohrreaktors, d.h. von Innenwand zu Innenwand.

Weiterer charakterisierender Parameter ist der Winkel α, wobei α den Winkel der Außenwand mit der Längsachse des Rohrreaktors beschreibt.

Ein positiver Winkel α beschreibt so also eine sich im Strömungsverlauf erweiternde Außenwand des Rohrreaktors, ein negativer Winkel α dagegen eine Verjüngung.

Durch den Parameter R wird die durchströmte Fläche F des Reaktors indirekt bestimmt, durch α deren Verlauf in Strömungsrichtung.

Es versteht sich von selbst, daß an den Übergängen von einem Abschnitt zum anderen die Werte von R übereinstimmen. Die Veränderungen der einzelnen Werte beziehen sich jeweils auf den Verlauf in den Abschnitten.

Gegenstand der vorliegenden Erfindung sind weiterhin verschiedene bevorzugte Konstruktionen des Reaktionsraums, die sich in der Größe der Querschnittsflächen entlang der Längsachse des Reaktors im Verlauf der Strömung unterscheiden:

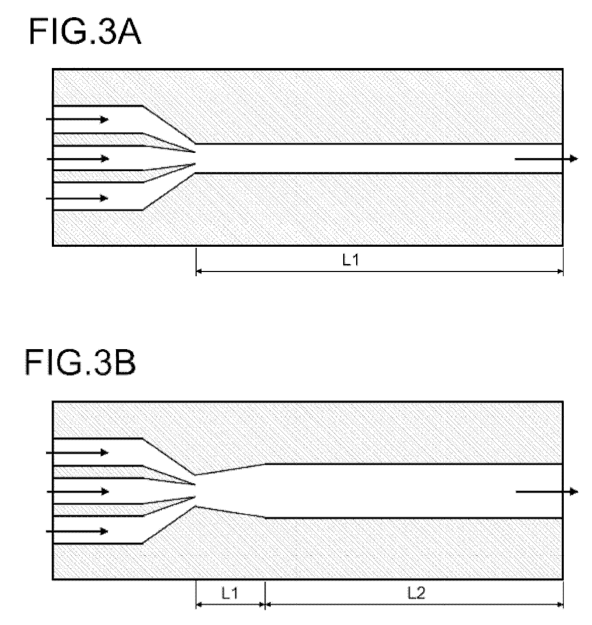

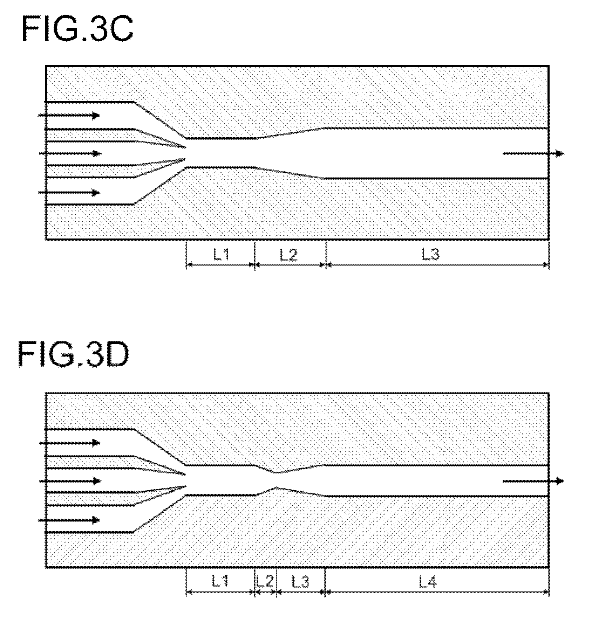

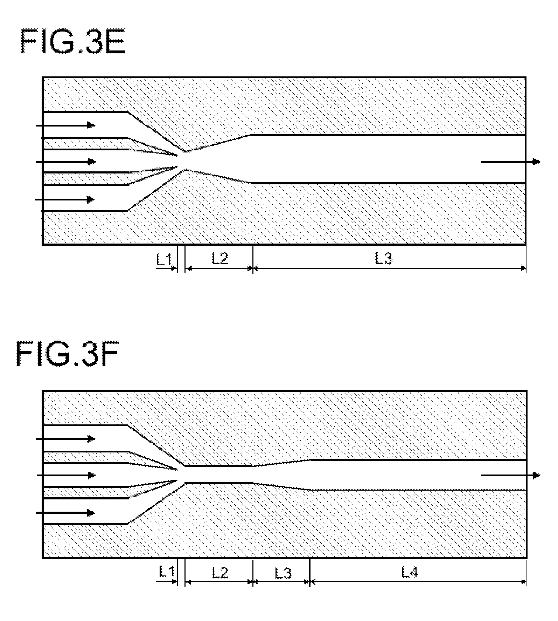

In der ersten Konstruktionsalternative (Figur 3, Darstellung a) ändert sich die durchströmte Fläche F im Verlauf des Reaktors nicht, d.h. es wird keine konstruktive Unterscheidung zwischen Abschnitten getroffen, stattdessen existiert lediglich ein einziger Abschnitt 1. In dessen Verlauf bleibt R₁ über die gesamte Länge des Reaktors gleich, α₁ ist 0°. Der gesamte Reaktor wird also aus einem Mischraum gebildet, bevorzugt aus einem rechteckigen Mischraum.

In einer zweiten Konstruktionsalternative (Figur 3, Darstellung b) vergrößert sich die durchströmte Fläche F₁ in einem Abschnitt 1 direkt nach Vereinigung der Ströme und bleibt danach in einem zweiten Abschnitt 2 konstant.

Dies kann dadurch erreicht werden, daß man R₁ vergrößert. In diesem Fall beträgt der Winkel α₁ von 0,0001 bis 10°, bevorzugt von 0,0002 bis 8 °, besonders bevorzugt von 0,0003 bis 7° und ganz besonders bevorzugt von 0,0003 bis 6 °.

Im Abschnitt 2 bleibt dann R₂ konstant, α₂ ist 0°.

In einer dritten Konstruktionsalternative (Figur 3, Darstellung c) bleibt die Fläche F₁ in einem Abschnitt 1 gleich und erweitert sich im zweiten Abschnitt um im dritten Abschnitt wieder konstant zu bleiben.

Dazu bleibt im Abschnitt 1 R₁ konstant, α₁ ist 0°.

Im zweiten Abschnitt vergrößert sich F₂, was durch ein anwachsende R₂ erreicht wird und bedeutet, daß α₂ beispielsweise von 0,0001 bis 10°, bevorzugt von 0,0002 bis 8 °, besonders bevorzugt von 0,0003 bis 7° und ganz besonders bevorzugt von 0,0003 bis 6 ° beträgt.

Im dritten Abschnitt bleibt F₃ konstant, dies wird dadurch erreicht, daß R₃ konstant und α₃ gleich 0° ist.

In einer vierten Konstruktionsalternative (Figur 3, Darstellung d) wird in einem Abschnitt 1 die Fläche F₁ gleichgehalten, im Abschnitt 2 wird F₂ dann verringert und in Abschnitt 3 F₃ vergrößert und im Abschnitt 4 konstant gehalten wird.

Dies wird dadurch erreicht, daß man R₁ konstant hält und α₁ auf 0° hält.

Im zweiten Abschnitt verringert sich F₂, was durch ein verkleinertes R₂ erreicht wird. Dazu beträgt α₂ beispielsweise weniger als 0° und mehr als -90°, bevorzugt -7 bis -65°, besonders bevorzugt -15 bis -35° und ganz besonders bevorzugt -20 bis -30°.

Im dritten Abschnitt vergrößert sich F₃, was erreicht wird durch ein anwachsende R₃ erreicht wird, was bedeutet, daß α₃ beispielsweise von 0,0001 bis 10°, bevorzugt von 0,0002 bis 8 °, besonders bevorzugt von 0,0003 bis 7° und ganz besonders bevorzugt von 0,0003 bis 6 ° beträgt.

Im vierten Abschnitt bleibt F₄ dann konstant, dazu bleibt im Abschnitt 4 R₄ konstant, α₄ ist 0°.

In einer fünften Konstruktionsalternative (Figur 3, Darstellung e) wird im ersten Abschnitt die Fläche F₁ verkleinert, im zweiten Abschnitt F₂ vergrößert und im dritten Abschnitt F₃ konstant gehalten.

Im ersten Abschnitt verringert sich F₁, was durch ein sich verkleinerndes R₁ erreicht wird. α₁ beträgt beispielsweise weniger als 0° und mehr als -90°, bevorzugt -7 bis -65°, besonders bevorzugt -15 bis -35° und ganz besonders bevorzugt -20 bis -30°.

Im zweiten Abschnitt vergrößert sich F₂, was erreicht wird durch ein anwachsendes R₂, was bedeutet, daß α₂ beispielsweise von 0,0001 bis 10°, bevorzugt von 0,0002 bis 8 °, besonders bevorzugt von 0,0003 bis 7° und ganz besonders bevorzugt von 0,0003 bis 6 ° beträgt.

Im Abschnitt 3 bleibt R₃ konstant, α₃ ist 0°.

In der sechsten Konstruktionsalternative (Figur 3, Darstellung f) wird die Fläche F₁ verringert, bleibt im Abschnitt zwei konstant, wird im Abschnitt 3 wieder vergrößert und bleibt im vierten Abschnitt dann wieder konstant.

Im ersten Abschnitt verringert sich F₁, was durch ein sich verkleinerndes R₁ erreicht wird. α₁ beträgt beispielsweise weniger als 0° und mehr als -90°, bevorzugt -7 bis -65°, besonders bevorzugt -15 bis -35° und ganz besonders bevorzugt -20 bis -30°.

Im Abschnitt 2 bleiben R₂ konstant, α₂ beträgt 0°.

Im dritten Abschnitt vergrößert sich F₃, was erreicht wird durch ein anwachsendes R₃, was bedeutet, daß α₃ beispielsweise von 0,0001 bis 10°, bevorzugt von 0,0002 bis 8 °, besonders bevorzugt von 0,0003 bis 7° und ganz besonders bevorzugt von 0,0003 bis 6 ° beträgt.

Im Abschnitt 4 bleibt R₄ konstant, α₄ beträgt 0°.

In der sechsten Konstruktionsalternative wird die Länge des Abschnitts 2 je nach eingesetztem Amin unterschiedlich gewählt: Bei stark ablagerungsbildenden Isocyanaten, wie z.B. (cyclo)aliphatischen Isocyanaten und insbesondere 1,6-Hexamethylendiisocyanat, wird ein kurzer Abschnitt 2 bevorzugt.

Die Länge der Zone 2, also L₂, ist in der Regel kleiner als das 30-fache der resultierenden Spaltbreite (R₂), bevorzugt kleiner als das 15-fache, besonders bevorzugt kleiner als das 10-fache, ganz besonders bevorzugt kleiner als das 6-fache, insbesondere kleiner als das fünffache und im speziellen kleiner als das dreifache. Oft ist L₂ kleiner als das zweifache und sogar kleiner als das einfache der Spaltbreite (R₂).

Hingegen wird bei wenig ablagerungsbildenden Isocyanaten, wie beispielsweise aromatischen isocyanaten und insbesondere Toluylendiisocyanat, ein möglichst große Länge des Abschnitts 2 bevorzugt.

Die Länge der Zone 2 ist bei derartigen Isocyanaten größer als das einfache der resulierenden Spaltbreite (R₂), bevorzugt größer als das 5-fache, besonders bevorzugt größer als das 10-fache, ganz besonders bevorzugt größer als das 15-fache und im speziellen größer als 30-fache.

Die Länge des jeweils letzten Abschnitts mit gleichbeliebender Fläche F, also in der sechsten Konstruktionsalternative L₄, wird so gewählt, dass man einerseits die gewünschte mittlere Kontaktzeit im Reaktor erreicht und andererseits ein L₄/(2 * R₄)-Verhältnis von größer 2, bevorzugt größer 4, besonders bevorzugt größer 6, ganz besonders bevorzugt größer 10 und im speziellen größer 15 erreicht.

In Abschnitten gleicher oder zunehmender Fläche wird F so gewählt, dass die mittlere Geschwindigkeit des Reaktionsgemischs im allgemeinen größer ist als 60 m/s, bevorzugt größer als 100, besonders bevorzugt größer als 160 m/s und ganz besonders bevorzugt größer als 180 m/s ist und im speziellen größer 200 m/s. Zudem wird F so gewählt, dass die mittlere Geschwindigkeit im allgemeinen kleiner ist als 250 m/s, bevorzugt kleiner als 240, besonders bevorzugt kleiner als 230 m/s, ganz besonders bevorzugt kleiner als 220 m/s und im speziellen kleiner als 210 m/s ist.

Die Veränderung des Parameters R kann innerhalb der Abschnitte linear oder nichtlinear erfolgen. Bei einer linearen Änderung bleibt der Winkel α innerhalb der Abschnitte gleich und verändert sich erst bei den Übergängen zwischen den Abschnitten. Bei nicht-linearem Verlauf innerhalb der Abschnitte kann der Verlauf konkav, d.h. in den Strömungsraum geneigt, konvex, d.h. vom Strömungsraum wegweisend, oder gemischt konkav-konvex bzw. konvex-konkav sein. Ein konkaver Verlauf bedeutet für den Winkel α, daß dieser sich im Verlauf der Strömung vergrößert bzw. positiver wird. Bevorzugt wird ein linearer Verlauf.

Die Übergänge zwischen den jeweiligen Abschnitten 1 bis 4 können unabhängig voneinander kantig oder abgerundet ausgeführt werden. Bevorzugt wird eine abgerundete Ausführung.

Die Konstruktionsalternativen sind als bevorzugte Ausführungsformen in Figur 3 (nicht maßstabsgerecht) wiedergegeben und analog der Numerierung in Figur 1a dargestellt. Besonders bevorzugt ist die Konstruktionsalternative gemäß Figur 3f:

Um den Spalt zur Eindosierung des Amins 2 sind zwei umgebende Spalte 1 zur Eindosierung von Phosgen angeordnet. Der Reaktionsraum 3/4 ist durch eine äußere Reaktorwand 5 begrenzt und entlang dem Verlauf der Reaktion in vier Abschnitte der Länge L₁, L₂, L₃ und L₄ aufgeteilt, die sich durch ihre durchströmte Fläche unterscheiden. Im Verlauf des ersten Abschnitts, direkt nach der Vermischung der Eduktströme, wird die durchströmte Fläche F₁ durch Verkleinerung von R₁ verringert, bleibt im Abschnitt 2 gleich, erweitert sich im Abschnitt 3 wieder durch Vergrößerung von R₃, um dann im vierten Abschnitt konstant zu bleiben.

Das durchströmte Volumen des Reaktors kann mit statischen Mischern ausgefüllt sein, beispielsweise Packungen, Formkörpern, Geweben, Loch- oder Schlitzblechen, bevorzugt ist das Volumen jedoch möglichst frei von Einbauten.

Denkbar ist auch der Einbau von Leitblechen in den Reaktionsraum. Ein geeignetes, turbulenzerzeugendes Element wäre beispielsweise ein eingelassenes spiralförmig verdrilltes Band, runde oder eckige Schrägplatten oder dergleichen.

Um auch bei großen Amin- und Phosgenmengenströmen, wie sie bei der Isocyanatproduktion im großtechnischen Maßstab üblich sind, kleine Mischungsweglängen und damit kurze Mischzeiten einzuhalten, bietet sich eine Parallelschaltung vieler kleiner Mischdüsen mit anschließender Misch- und Reaktionszone an, wobei die parallelgeschalteten Einheiten durch Wandungen von einander getrennt sind. Der Vorteil dieser Verfahrensvariante liegt in einem günstigeren Längen- zu Durchmesserverhältnis der Misch- und Reaktionszonen. Je größer dieses Verhältnis ist, desto günstiger (enger) ist die Verweilzeitverteilung der Strömung. Somit kann bei gleicher Verweilzeit und Strömungsgeschwindigkeit durch viele parallelgeschaltete Einheiten, dass Längen- zu Durchmesserverhältnis erhöht werden und damit auch die Verweilzeitverteilung eingeengt werden. Um den apparativen Aufwand gering zuhalten, münden die einzelnen Reaktionszonen in eine gemeinsame Nachreaktionszone, in der dann der Restumsatz des Amins erfolgt.

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Hexan, Benzol, Nitrobenzol, Anisol, Chlorbenzol, Chlortoluol, o-Dichlorbenzol, Trichlorbenzol, Diethylisophthalat (DEIP), Tetrahydrofuran (THF), Dimethylformamid (DMF), Xylol, Chlornaphthalin, Decahydronaphthalin und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamylchlorids im gewählten Quenchmedium gehalten. Besonders bevorzugt wird dabei die Temperatur des inerten Lösungsmittels oberhalb der Schmelztemperatur des zum Amin gehörigen Carbamylchlorids gehalten

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Ein geeigneter Quench ist beispielsweise bekannt aus EP-A1 1403248, Sp. 2, Z. 39 - Sp. 3, Z. 18, auf die im Umfang dieser Offenbarung ausdrücklich Bezug genommen sei.

In dieser Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 140 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der internationalen Anmeldung WO 2005/123665 beschrieben.

Die Flüssigkeitströpfchen werden dazu mittels Ein- oder Zweistoffzerstäuberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können.

In einer besonderen Ausführungsform des Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie HCl und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.

In der anschließenden optionalen Reinigungsstufe wird das Isocyanat, bevorzugt durch Destillation, vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A1 10260092.

In einer besonderen Ausführungsform des erfindungsgemäßen Verfahrens werden die phosgenhaltigen und die aminhaltigen Eduktströme jeweils über mindestens eine schlitzförmige Öffnung der Mischzone zugeführt. Bevorzugt werden die Phosgen- und Amin führenden Öffnungen alternierend angeordnet. Bevorzugt sind die Aminspalte so angeordnet, dass zwischen ihnen und der Mischzonenwand mindestens eine phosgenführende Öffnung liegt. Die Amin- und Phosgenspalte können eine konstante oder aber auch eine variable Breite aufweisen. Ferner können die gedachten Mittellinien der Spalte beliebige Kurven beschreiben. Bevorzugt beschreiben sie Kreise, Dreiecke oder allgemein Vielecke, Ellipsen oder Geraden. Die Figuren 1 a und 1 b zeigen eine ebene Ausführungsform der Mischdüse, bei welcher der durch einen geraden Spalt zugeführte Aminstrom durch einen umlaufenden Phosgenhüllstrom ummantelt ist, so dass ein vorzeitiger Kontakt von aminreichem Reaktionsgemisch mit der Wand vermieden wird. In der Mischzone erfolgt eine intensive Vermischung der Edukte. Der Mischzone ist dann eine Reaktionszone nachgeschaltet.

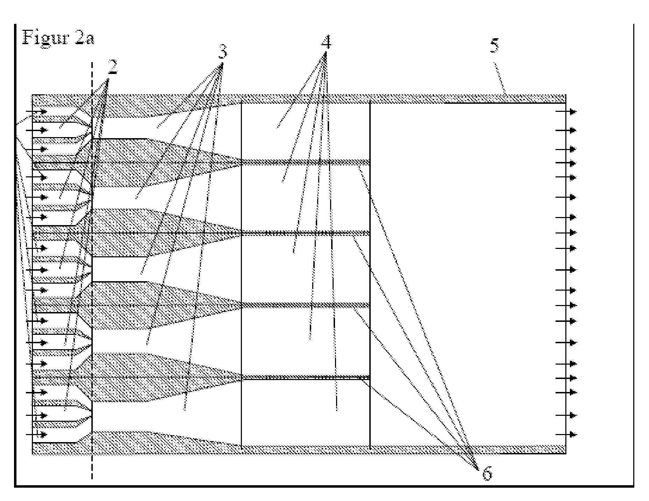

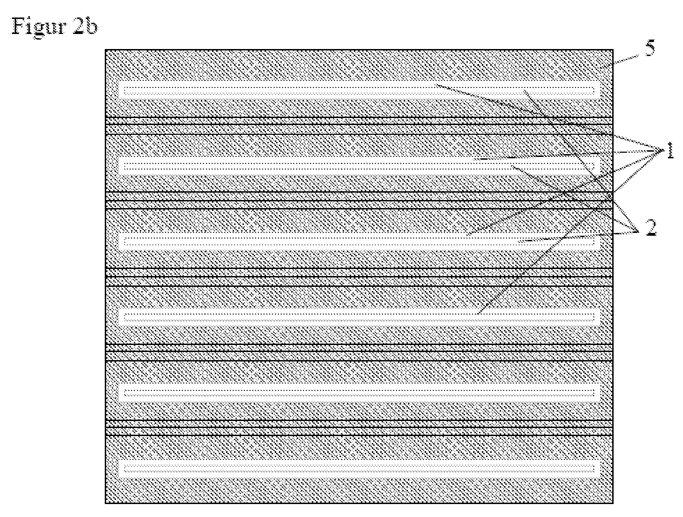

In einer weiteren bevorzugten Ausführungsform des erfindungsgemäßen Verfahrens wird eine Vielzahl von Mischdüsen parallel geschaltet oder gestapelt. Dabei gehört zu jeder Mischdüse mindestens eine Amin- und eine Phosgenzuführung. Zu jeder Mischdüse gehört eine Mischzone und Reaktionszone, wobei die einzelnen Misch- und die Reaktionszonen voneinander durch Wandungen getrennt sind. Die einzelnen Reaktionszonen münden dann in eine gemeinsame Nachreaktionszone, in der dann der Restumsatz des Amins erreicht wird. Die Amin- und Phosgenzuführungen können beliebige Formen aufweisen. So können die Zuführungen beispielweise kreisförmig, elliptisch, dreieckig, viereckig oder allgemein vieleckig oder aber spaltförmig sein, wobei die gedachte Spaltmittellinie eine beliebige Form besitzen kann. Bevorzugt werden schlitzförmige Öffnungen gewählt. Die Abbildungen 2a und 2b zeigen die Misch- und Reaktionszone eines erfindungsgemäßen Verfahrens, bei dem die Vermischung von Amin- und Phosgen über eine Vielzahl von gestapelten Einzeldüsen erreicht wird. Jeder Einzeldüse ist jeweils eine Mischzone und eine Reaktionszone nachgeschaltet. Die Mischzonen sind untereinander durch Wandungen getrennt. Ebenso sind die Reaktionszonen durch Wandungen getrennt. Stromab von den einzelnen Reaktionszonen befindet sich eine gemeinsame Nachreaktionszone

In den Figuren 1a, 1 b, 2a und 2b haben die Bezugszeichen die folgende Bedeutung:
1: Phosgenzuführung
2: Aminzuführung
3: Mischzone
4: Reaktionszone
5: Reaktorwandungen
6: Trennwand

## Patentansprüche

1. Verfahren zur Herstellung von Isocyanaten durch Umsetzung der korrespondierenden Amine mit Phosgen, gegebenenfalls in Gegenwart von mindestens einem Inertmedium, in der Gasphase, **dadurch gekennzeichnet, dass** man in einem Reaktor n Aminströme mit mindestens n Phosgenströmen umsetzt, wobei n eine positive ganze Zahl von mindestens 1 ist, und man mindestens einen Amin- oder Phosgenstrom über mindestens einen Schlitz zur Vermischung in den Reaktor dosiert, wobei
man das Amin durch einen Schlitz dosiert, der an beiden Längsseiten von Schlitzen flankiert ist, durch die man Phosgen dosiert,
oder
durch einen inneren Schlitz Amin dosiert, der allseitig von einem zusammenhängenden Schlitz umgeben ist, durch den man Phosgen dosiert und wobei die Flächenform sämtlicher Schlitze rechteckig ist.

2. Verfahren nach Anspruch 1, **dadurch gekennzeichnet, dass** das Isocyanat ausgewählt ist aus der Gruppe bestehend aus 1,6-Diisocyanatohexan, 1-Isocyanato-3,3,5- trimethyl-5-(isocyanatomethyl)cyclohexan, 4,4'-Di(isocyanatocyclo-hexyl)methan und Toluylendiisocyanat-Isomerengemischen,

3. Verfahren nach einem der vorstehenden Ansprüche, **dadurch gekennzeichnet, dass** mindestens ein Inertmedium anwesend ist, ausgewählt aus der Gruppe bestehend aus Stickstoff, Helium, Argon, Chlorbenzol, Chlortoluol, o-Dichlorbenzol, Toluol, Xylol, Chlornaphthalin, Decahydronaphthalin, Kohlenstoffdioxid und Kohlenstoffmonoxid.

4. Verfahren gemäß Anspruch 3, **dadurch gekennzeichnet, dass** das Gasvolumen von Inertmedium zu Amin bzw. zu Phosgen mehr als 0,0001 bis 30 beträgt.

5. Verfahren nach einem der vorstehenden Ansprüche, **dadurch gekennzeichnet, dass** die Temperatur im Verfahren von 200 bis 600 °C beträgt.

6. Verfahren nach einem der vorstehenden Ansprüche, **dadurch gekennzeichnet, dass** man ein molares Verhältnis von Phosgen zu Aminogruppen von 1,1 : 1 bis 20 : 1 einstellt.

7. Verfahren nach einem der vorstehenden Ansprüche, **dadurch gekennzeichnet, dass** man als äußersten Strom an der Außenwand einen Phosgenstrom in den Reaktor dosiert.

8. Verfahren nach einem der vorstehenden Ansprüche, **dadurch gekennzeichnet, dass** das Verhältnis der Gesamtfläche der Aminströme zur Gesamtfläche der Phosgenströme größer als 0,00002 und kleiner als 5 ist.

9. Verfahren nach einem der vorstehenden Ansprüche, **dadurch gekennzeichnet, dass** man die einzelnen Edukte in der Mischeinrichtung mit einer Strömungsgeschwindigkeit von 20 bis 400 Meter/Sekunde in den Reaktor führt.

10. Verfahren nach Anspruch 1, **dadurch gekennzeichnet, dass** man durch n Schlitze Amin dosiert und durch (n + 1) Schlitze Phosgen dosiert, wobei jeder der n Schlitze, durch die man Amin dosiert, von jeweils 2 Schlitzen flankiert ist, durch die man Phosgen dosiert.

11. Verfahren nach Anspruch 1, **dadurch gekennzeichnet, dass** man durch einen inneren Schlitz Amin dosiert, der allseitig von einem zusammenhängenden Schlitz umgeben ist, durch den man Phosgen dosiert und wobei der äußere Schlitz, durch den man Phosgen dosiert, von einem weiteren zusammenhängenden oder nicht-zusammenhängenden Schlitz zur Amindosierung flankiert ist, der wiederum allseitig von einem zusammenhängenden Schlitz umgeben ist, durch den man Phosgen dosiert.

12. Verfahren nach einem der vorstehenden Ansprüche, **dadurch gekennzeichnet, dass** Mischzeit des phosgenhaltigen mit dem aminhaltigen Eduktstrom weniger als 10 ms beträgt.

13. Verfahren nach einem der vorstehenden Ansprüche, **dadurch gekennzeichnet, dass** die mittlere Kontaktzeit des Umsetzungsgemisches zwischen 0,001 Sekunden und weniger als 5 Sekunden beträgt.

14. Verfahren nach einem der vorstehenden Ansprüche, **dadurch gekennzeichnet, daß** die Strömung im Reaktor eine Bodenstein-Zahl von mehr als 10 aufweist.

15. Verfahren nach einem der vorstehenden Ansprüche, **dadurch gekennzeichnet, dass** die Strömung des Reaktionsgemischs eine Reynolds-Zahl von mindestens 2.300 aufweist.

16. Verfahren nach einem der vorstehenden Ansprüche, **dadurch gekennzeichnet, dass** der Umsatz an Amin in dem Raum, in dem die Vermischung der Edukte zu einem Grad von 99 % stattfindet, nicht mehr als 15 % beträgt.

## Claims

1. A process for preparing isocyanates by reacting the corresponding amines with phosgene, optionally in the presence of at least one inert medium, in the gas phase, which comprises reacting n amine streams with at least n phosgene streams, where n is a positive integer of at least 1, in a reactor and introducing at least one amine or phosgene stream into the reactor via at least one slit for mixing,
where
the amine is introduced through a slit which is flanked on both longitudinal sides by slits through which phosgene is introduced
or amine is introduced through an inner slit which is surrounded on all sides by a contiguous slit through which phosgene is introduced and the two-dimensional shape of all the slits is rectangular.

2. The process according to claim 1, wherein the isocyanate is selected from the group consisting of 1,6-diisocyanatohexane, 1-isocyanato-3,3,5-trimethyl-5-(isocyanatomethyl)cyclohexane, 4,4'-di(isocyanatocyclohexyl)methane and tolylene diisocyanate isomer mixtures.

3. The process according to either of the preceding claims, wherein at least one inert medium selected from the group consisting of nitrogen, helium, argon, chlorobenzene, chlorotoluene, o-dichlorobenzene, toluene, xylene, chloronaphthalene, decahydronaphthalene, carbon dioxide and carbon monoxide is present.

4. The process according to claim 3, wherein the gas volume ratio of inert medium to amine or to phosgene is more than 0.0001 to 30.

5. The process according to any of the preceding claims, wherein the temperature in the process is from 200 to 600°C.

6. The process according to any of the preceding claims, wherein a molar ratio of phosgene to amino groups of from 1.1:1 to 20:1 is set.

7. The process according to any of the preceding claims, wherein a phosgene stream is introduced into the reactor as outermost stream at the outer wall.

8. The process according to any of the preceding claims, wherein the ratio of the total area of the amine streams to the total area of the phosgene streams is greater than 0.00002 and less than 5.

9. The process according to any of the preceding claims, wherein the individual starting materials in the mixing device are fed into the reactor at a flow velocity of from 20 to 400 meter/second.

10. The process according to claim 1, wherein amine is introduced through n slits and phosgene is introduced through (n+1) slits, with each of the n slits through which amine is introduced being flanked by 2 slits through which phosgene is introduced.

11. The process according to claim 1, wherein amine is introduced through an inner slit which is surrounded on all sides by a contiguous slit through which phosgene is introduced and the outer slit through which phosgene is introduced is flanked by a further contiguous or noncontiguous slit for introduction of amine and this is in turn surrounded on all sides by a contiguous slit through which phosgene is introduced.

12. The process according to any of the preceding claims, wherein the mixing time of the phosgene-comprising feed stream with the amine-comprising feed stream is less than 10 ms.

13. The process according to any of the preceding claims, wherein the mean contact time of the reaction mixture is from 0.001 second to < 5 seconds.

14. The process according to any of the preceding claims, wherein the flow in the reactor has a Bodenstein number of more than 10.

15. The process according to any of the preceding claims, wherein the flow of the reaction mixture has a Reynolds number of at least 2300.

16. The process according to any of the preceding claims, wherein the conversion of amine in the space in which mixing of the starting materials takes place to an extent of 99% is not more than 15%.

## Revendications

1. Procédé de fabrication d'isocyanates par mise en réaction des amines correspondantes avec du phosgène, éventuellement en présence d'au moins un milieu inerte, dans la phase gazeuse, **caractérisé en ce que** n courants d'amine sont mis en réaction dans un réacteur avec au moins n courants de phosgène, n étant un nombre entier positif d'au moins 1 et au moins un courant d'amine ou de phosgène étant introduit dans le réacteur par au moins une fente pour le mélange,
l'amine étant introduite par une fente, qui est flanquée sur les deux côtés longitudinaux par des fentes par lesquelles le phosgène est introduit,
ou
l'amine étant introduite par une fente intérieure, qui est entourée sur tous les côtés par une fente continue par laquelle le phosgène est introduit, la forme de surface de toutes les fentes étant rectangulaire.

2. Procédé selon la revendication 1, **caractérisée en ce que** l'isocyanate est choisi dans le groupe constitué par le 1,6-diisocyanatohexane, le 1-isocyanato-3,3,5-triméthyl-5-(isocyanatométhyl)cyclohexane, le 4,4'-di(isocyanatocyclohexyl)méthane et le mélange d'isomères de diisocyanate de toluylène.

3. Procédé selon l'une quelconque des revendications précédentes, **caractérisé en ce qu'**au moins un milieu inerte est présent, choisi dans le groupe constitué par l'azote, l'hélium, l'argon, le chlorobenzène, le chlorotoluène, l'o-dichlorobenzène, le toluène, le xylène, la chloronaphtaline, la décahydronaphtaline, le dioxyde de carbone et le monoxyde de carbone.

4. Procédé selon la revendication 3, **caractérisé en ce que** le volume gazeux du milieu inerte par rapport à l'amine ou au phosgène est de plus de 0,0001 à 30.

5. Procédé selon l'une quelconque des revendications précédentes, **caractérisé en ce que** la température lors du procédé est de 200 à 600 °C.

6. Procédé selon l'une quelconque des revendications précédentes, **caractérisé en ce qu'**un rapport molaire entre le phosgène et les groupes amino de 1,1:1 à 20:1 est ajusté.

7. Procédé selon l'une quelconque des revendications précédentes, **caractérisé en ce qu'**un courant de phosgène est introduit dans le réacteur sous la forme du courant le plus extérieur sur la paroi extérieure.

8. Procédé selon l'une quelconque des revendications précédentes, **caractérisé en ce que** le rapport entre la surface totale des courants d'amine et la surface totale des courants de phosgène est supérieur à 0,00002 et inférieur à 5.

9. Procédé selon l'une quelconque des revendications précédentes, **caractérisé en ce que** les réactifs individuels sont introduits dans l'unité de mélange dans le réacteur à une vitesse d'écoulement de 20 à 400 mètres/seconde.

10. Procédé selon la revendication 1, **caractérisé en ce que** l'amine est introduite par n fentes et le phosgène est introduit par (n + 1) fentes, chacune des n fentes par lesquelles l'amine est introduite étant flanquée par 2 fentes par lesquelles le phosgène est introduit.

11. Procédé selon la revendication 1, **caractérisé en ce que** l'amine est introduite par une fente intérieure, qui est entourée sur tous les côtés par une fente continue par laquelle le phosgène est introduit, la fente extérieure par laquelle le phosgène est introduit étant flanqué par une fente continue ou non continue supplémentaire pour l'introduction de l'amine, qui est de nouveau entourée sur tous les côtés par une fente continue par laquelle le phosgène est introduit.

12. Procédé selon l'une quelconque des revendications précédentes, **caractérisé en ce que** la durée de mélange du courant de réactif contenant le phosgène avec le courant de réactif contenant l'amine est de moins de 10 ms.

13. Procédé selon l'une quelconque des revendications précédentes, **caractérisé en ce que** la durée de contact moyenne du mélange de réaction est comprise entre 0,001 seconde et moins de 5 secondes.

14. Procédé selon l'une quelconque des revendications précédentes, **caractérisé en ce que** l'écoulement dans le réacteur présente un nombre de Bodenstein de plus de 10.

15. Procédé selon l'une quelconque des revendications précédentes, **caractérisé en ce que** l'écoulement du mélange réactionnel présente un nombre de Reynolds d'au moins 2 300.

16. Procédé selon l'une quelconque des revendications précédentes, **caractérisé en ce que** la conversion de l'amine dans l'espace où le mélange des réactifs a lieu à un degré de 99 % est inférieure ou égale à 15 %.
